(19) Europäisches Patentamt
European Patent Office
Office européen des brevets

(11) Veröffentlichungsnummer: **0 022 086**
**A1**

(12) # EUROPÄISCHE PATENTANMELDUNG

(21) Anmeldenummer: **80810208.1**

(22) Anmeldetag: **23.06.80**

(51) Int. Cl.³: **C 07 D 263/52**
C 07 D 498/10, A 01 N 43/76
A 01 N 43/90
//C07C103/737, C07F7/18,
C07D307/93, (C07D498/10, 307/00,
263/00)

(30) Priorität: **27.06.79 CH 5995/79**
**03.06.80 CH 4285/80**

(43) Veröffentlichungstag der Anmeldung:
**07.01.81 Patentblatt 81/1**

(84) Benannte Vertragsstaaten:
**AT BE CH DE FR GB IT LI NL**

(71) Anmelder: **CIBA-GEIGY AG**
**Patentabteilung Postfach**
**CH-4002 Basel(CH)**

(72) Erfinder: **Eckhardt, Wolfgang, Dr.**
**Breslauerstrasse 16**
**D-7850 Lörrach(DE)**

(54) **4-Oxa-6-aza-6(phenyl)-spiro(2.4)heptan-5,7-dione, Herstellung und Verwendung als Mikrobizide.**

(57) Verbindungen der Formel I

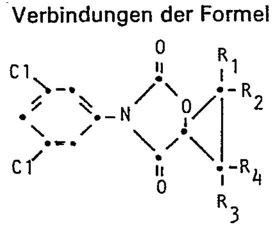

(1)

worin mindestens einer der Substituenten $R_1$, $R_2$, $R_3$, $R_4$ $C_1$-$C_4$-Alkyl ist und die anderen unabhängig voneinander Wasserstoff oder $C_1$-$C_4$-Alkyl bedeuten oder, falls $R_1$ und $R_3$ gleichzeitig Wasserstoff bedeuten, $R_2$ und $R_4$ gegebenenfalls eine $C_2$-$C_4$-Alkylenbrücke bilden, in der ein $CH_2$-Glied auch durch Sauerstoff ersetzt sein kann.

Diese Verbindungen weisen wertvolle mikrobizide Eigenschaften auf. Sie lassen sich allein oder in Form von Schädlingsbekämpfungsmitteln in der Praxis anwenden, vor allem zum Schutz von Kulturpflanzen vor Pilzbefall. Verbindungen der Formel I wirken sowohl residual-protektiv als auch systemisch.

EP 0 022 086 A1

Croydon Printing Company Ltd.

CIBA-GEIGY AG
Basel (Schweiz)

5-12406/1+2/=

# 4-Oxa-6-aza-6(phenyl)-spiro[2.4]heptan-5,7-dione, Herstellung und Verwendung als Mikrobizide

Die vorliegende Erfindung betrifft eine Verbindung der Formel I

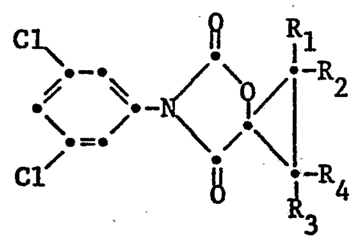

(I)

worin mindestens einer der Substituenten $R_1$, $R_2$, $R_3$, $R_4$ $C_1$-$C_4$-Alkyl ist und die anderen unabhängig voneinander Wasserstoff oder $C_1$-$C_4$-Alkyl bedeuten oder, falls $R_1$ und $R_3$ gleichzeitig Wasserstoff bedeuten, $R_2$ und $R_4$ gegebenenfalls eine $C_2$-$C_4$-Alkylenbrücke bilden, in der ein $CH_2$-Glied auch durch Sauerstoff ersetzt sein kann.

Unter Alkyl oder als Alkylteil eines Substituenten sind je nach Zahl der angegebenen Kohlenstoffatome folgende Gruppen zu verstehen: Methyl, Aethyl, Propyl, Butyl, iso-Butyl, sek.-Butyl, tert.-Butyl. Der Begriff $C_2$-$C_4$ Alkylenbrücke steht entweder für eine Aethylen-, Propylen- oder Butylenbrücke.

Mit dieser beispielhaften Aufzählung ist keine Limitierung verbunden.

Die Verbindungen der Formel I liegen als Diastereomerenpaare vor. Die Auftrennung in die einzelnen Isomeren kann auf übliche Art, vorzugsweise beim unten genannten Edukt III oder beim Zwischenprodukt IV vorgenommen werden.

Sofern im folgenden keine abweichenden Angaben gemacht werden, handelt es sich bei den angegebenen Verbindungen der Formel I um Isomerengemische.

Verbindungen der Formel I besitzen mikrobizide Wirkung.

Die Verbindungen der Formel I können nach einer ganzen Reihe von Methoden hergestellt werden. In den Formeln II bis XI haben $R_1$ bis $R_4$ die unter Formel I angegebenen Bedeutungen, M steht für ein Metall-, vorzugsweise Alkalimetallkation, wie z.B. Natrium oder Kalium, und R für Alkyl.

Verbindungen der Formel I können z.B. nach einem der folgenden Formelschemata A bis E erhalten werden, indem man in an sich bekannter Art, z.B. bei Raumtemperatur Verbindungen der Formel III, worin R für Wasserstoff oder Alkyl steht, mit Isocyanaten der Formel II umsetzt und dann die Zwischenprodukte der Formel IV vorzugsweise bei erhöhten Temperaturen, gegebenenfalls in Anwesenheit eines Kondensationsmittels zu Verbindungen der Formel I cyclisiert;

A.

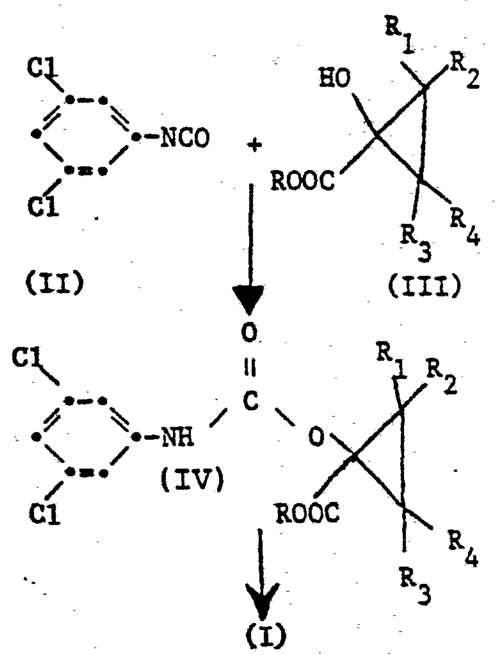

(II) (III)

(IV)

(I)

B. oder indem man

(II) + (V)

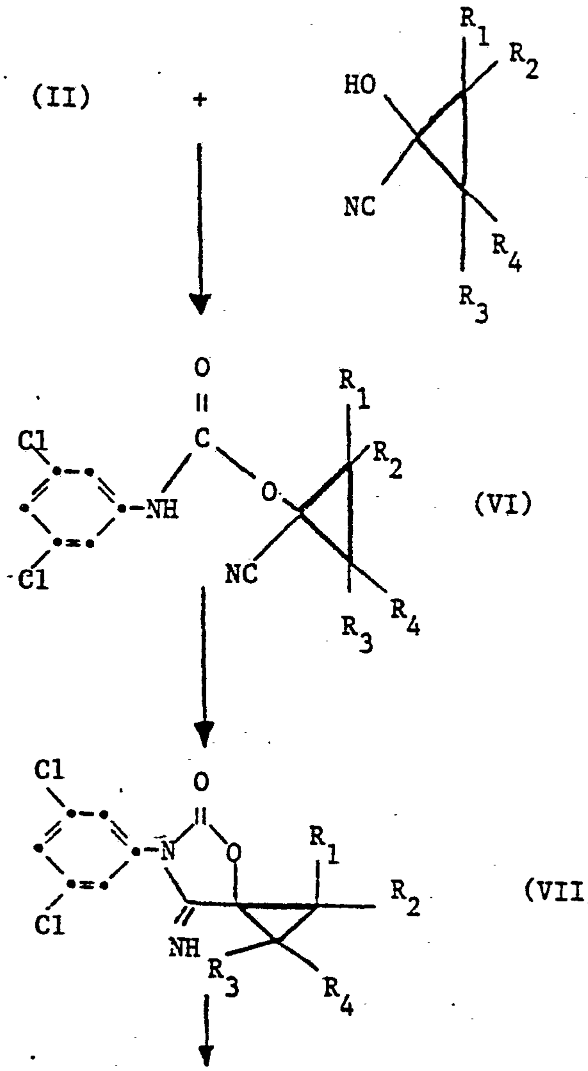

(VI)

(VII)

(I)

vorzugsweise bei Raumtemperatur Verbindungen der Formel V mit Isocyanaten der Formel II umsetzt und die Zwischenprodukte vorzugsweise bei erhöhten Temperaturen und gegebenenfalls bei Anwesenheit eines Kondensationsmittels zu Verbindungen der Formel VIII cyclisiert und diese durch saure Hydrolyse in die Endprodukte der Formel I überführt;

C. oder indem man

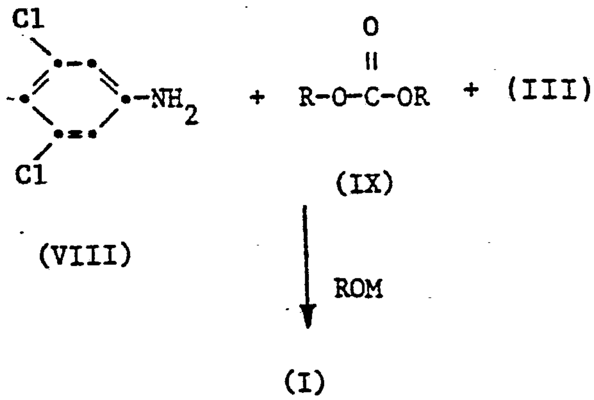


(VIII) (IX) (III) (I)

vorzugsweise in Anwesenheit von Alkoholaten, Verbindungen der Formel III und IX mit substituierten Anilinen der Formel VIII zu Produkten der Formel I kondensiert;

D. oder indem man

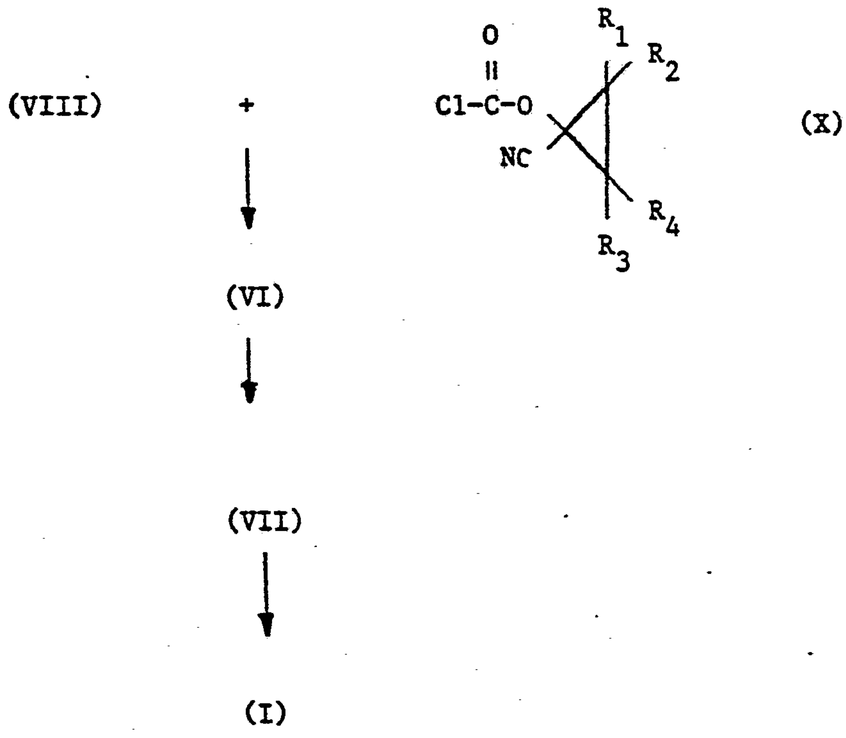

(VIII) (X) (VI) (VII) (I)

substituierte Aniline der Formel VIII mit Verbindungen der Formel X, vorzugsweise in Anwesenheit von säurebindenen Mitteln zu Zwischenprodukten der Formel VI umsetzt und diese, wie bei Verfahren B beschrieben, in die Endprodukte der Formel I überführt.

E. oder indem man

(VIII)

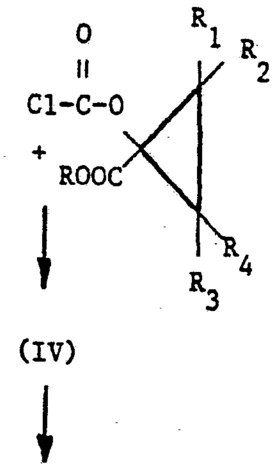

(XI)

(IV)

(I).

substituierte Aniline der Formel VIII mit Verbindungen der Formel XI, vorzugsweise in Anwesenheit eines säurebindenden Mittels zu den Zwischenprodukten der Formel IV kondensiert und letztere, wie bei Verfahren A beschrieben, in die Endprodukte der Formel I überführt.

Es ist bei allen Verfahren zweckmässig, die Reaktion in einem Lösungsmittel durchzuführen, das gegenüber den Reaktionsteilnehmern inert ist. Das Verfahren kann auch ohne Anwesenheit eines Lösungsmittels durchgeführt werden.

Beispiele für solche Lösungsmittel sind:

Aromatische und aliphatische Kohlenwasserstoffe wie Benzol, Toluol, Xylole, Petroläther, Ligroin, Cyclohexan; halogenierte Kohlenwasserstoffe wie Chlorbenzol, Methylenchlorid, Aethylenchlorid, Chloroform, Tetrachlorkohlenstoff, Tetrachloräthylen; Aether und ätherartige Verbindungen wie Diäthyläther, Diisopropyläther, t-Butylmethyläther, Dimethoxyäthan, Dioxan, Tetrahydrofuran, Anisol; Nitrile wie Acetonitril ; Ketone wie Aceton, Methyläthylketon; Ester wie Essigsäureäthylester, Butylacetat; Sulfone wie Dimethylsulfoxid und Gemische solcher Lösungsmittel untereinander.

Bei den beschriebenen Herstellungsverfahren ist es zweckmässig, freiwerdende Säuren durch entsprechende Bindemittel abzufangen. Hierzu eignen sich z.B. organische Basen wie Trialkylamine (z.B. Triäthylamin), Pyridin und Pyridinbasen oder anorganische Basen wie Oxide, Hydroxide, Hydrogencarbonate, Carbonate oder Hydride von Alkali- und Erdalkalimetallen sowie Natriumacetat. In den Fällen, wo Wasser freigesetzt wird, können als Bindemittel auch Carbonsäureanhydride wie Essigsäureanhydrid oder Mineralsäuren wie Halogenwasserstoffsäuren, Schwefelsäure, Phosphorsäure oder Cyclohexylcarbodiimid verwendet werden. Das Bindemittel wird dabei mindestens in molarer Menge, bezogen auf die freiwerdende Säure bzw. das freiwerdende Wasser, zugegeben.

Zur Beschleunigung des in den Herstellungsverfahren beschriebenen Cyclisierungsschrittes zu den entsprechenden 4-Oxa-6-aza-spiro[2.4]heptan-5,7-dionen arbeitet man zweckmässigerweise bei Temperaturen von -10° bis 160°C, vorzugsweise 20-100°C, bzw. am Siedepunkt des Lösungsmittels oder Lösungsmittelgemisches.

Bei den angegebenen Verfahren ist es nicht unbedingt erforderlich, die Zwischenprodukte der Formel IV, VI und VII zu isolieren. Die Herstellung der Endprodukte kann auch im Eintopfverfahren durchgeführt werden.

Die verschiedenen Verfahren sind Teil der Erfindung.

Alle Ausgangsstoffe werden nach an sich bekannten Methoden dargestellt. Die Verbindungen der Formeln II, VIII und IX sind bekannt; 1-Hydroxy-cyclopropan-1-carbonsäuren der Formel III werden in der DE-Offenlegunsschrift 2,128,327 beschrieben und lassen sich auf die übliche Art in die Derivate der Formeln V, X und XI überführen. Vgl. auch Liebigs Ann.Chem. 1976, S. 463-475. Von den Ausgangsstoffen wird keine biologische Wirkung berichtet.

3-(Phenyl)-oxazolidin-Derivate werden erwähnt in:

J.Org.Chem. 32, No. 2, 383-88
Tetrahedron 26, No. 16, 3875-82 (1970)
Agr. Biol. Chem. 35, No. 11, 1707-19 (1971)

Es wurde überraschenderweise gefunden, dass Verbindungen mit der Struktur der Formel I ein für praktische Bedürfnissse sehr viel günstigeres Mikrobizid-Spektrum als die 3-(3',5'-Dichlorphenyl)-oxazolidin-2,4-dione der Deutschen Offenlegungsschrift 1,811,843 aufweisen.

Als Beispiele für Kulturpflanzen seien im Rahmen der vorliegenden Erfindung genannt:
Getreide, Mais, Reis, Gemüse, Soja, Erdnüsse, Bohnen, Obstbäume, Beerenobst, Zierpflanzen, vor allem aber Reben, Hopfen, Gurkengewächse (z.B. Gurken, Kürbis, Melonen), Solanaceen wie Kartoffeln, Tabak und Tomaten sowie auch Bananen-, Kakao- und Naturkautschukgewächse.

Die Verbindungen der Formel I können für sich allein oder zusammen mit geeigneten Trägern und/oder anderen Zuschlagstoffen verwendet werden. Geeignete Träger und Zuschlagstoffe können fest oder flüssig sein und entsprechen den in der Formulierungstechnik üblichen Stoffen, wie z.B. natürlichen oder regenerierten mineralischen Stoffen, Lösungs-, Dispergier-, Netz-, Haft-, Verdickungs-, Bindeoder Düngemitteln.

Der Gehalt an Wirkstoff in handelsfähigen Mitteln liegt zwischen 0,1 bis 90%.

Zur Applikation können die Verbindungen der Formel I in den folgenden Aufarbeitungsformen vorliegen (wobei die Gewichts-Prozentangaben in Klammern vorteilhafte Mengen an Wirkstoff darstellen):

Feste Aufarbeitungsformen: Stäubemittel und Streumittel(bis zu 10%) Granulate, Umhüllungsgranulate, Imprägnierungsgranulate und Homogengranulate, Pellets (Körner) (1 bis 80%);

Flüssige Aufarbeitungsformen:

a) in Wasser dispergierbare Wirkstoffkonzentrate:
Spritzpulver (wettable powders) und Pasten (25-90% in der Handelspackung, 0,01 bis 15% in gebrauchsfertiger Lösung); Emulsions- und Lösungskonzentrate (10 bis 50%; 0,01 bis 15% in gebrauchsfertiger Lösung);

b) Lösungen (0,1 bis 20%);Aerosole.

Solche Mittel gehören ebenfalls zur Erfindung.

Mit Wirkstoffen der Formel I können an Pflanzen oder Pflanzenteilen (Früchte, Blüten, Laubwerk, Stengel, Knollen, Wurzeln) dieser und verwandter Nutzkulturen die auftretenden Mikroorganismen eingedämmt oder vernichtet werden, wobei auch später zuwachsende Pflanzenteile von derartigen Pilzen und Milben verschont bleiben. Die Wirkstoffe der Formel I sind gegen die den folgenden Klassen angehörenden phytopathogenen Pilzen wirksam: Fungi imperfecti (z.B. Botrytis), Ascomyceten wie Erysiphe, Rostpilze wie Puccinia- und Rhizoctonia-Erreger.

Wirkstoffe der Formel I wirken überdies systemisch und können ferner als Beizmittel zur Behandlung von Saatgut (Früchten, Knollen, Körner) und Pflanzenstecklingen zum Schutz von Pilzinfektion sowie gegen im Erdboden auftretende Mikroorganismen eingesetzt werden.

Die Erfindung betrifft somit ferner die Verwendung der Verbindungen der Formel I zur Bekämpfung phytopathogener Mikroorganismen.

Zu den bevorzugten Verbindungen gehören solche im Umfang der

Formel I, worin $R_1$ bis $R_4$ Wasserstoff oder Methyl bedeuten.

Besonders bevorzugt sind die weiter unten genannten Verbindungen Nr. 1, 2, 3 und 15.

Die nachfolgenden Beispiele dienen zur näheren Erläuterung der Erfindung, ohne diese jedoch einzuschränken. Temperaturangaben beziehen sich immer auf Celsiusgrade, Druckangaben auf Millibar und Teile stets auf Gewichtsteile.

Beispiel 1:

a) Herstellung des Zwischenproduktes

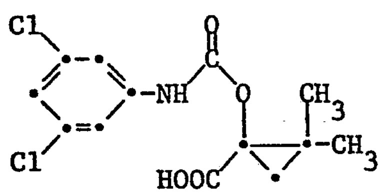

1-[N-(3',5'-dichlorphenyl)-carbonyloxy]-1-[(1,1-dimethyl)-cyclopropancarbonsäure

94 g (0,5 Mol) 3,5-Dichlorphenylisocyanat wurden in 500 ml Toluol gelöst und 65 g (0,5 Mol) 1-Hydroxy-1-(2,2-dimethyl)-cyclopropancarbonsäure bei Raumtemperatur zugegeben. Dann wurde 16 Stunden bei Raumtemperatur gerührt und anschliessend filtriert. Nach dem Trocknen verblieben 144 g Produkt vom Smp. 175-176°C.

b) Herstellung des Endproduktes

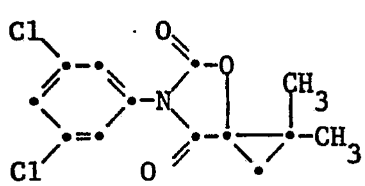

Verb. Nr. 2

4-oxa-6-aza-6(3',5'-dichlorphenyl)-1,1-dimethyl-spiro[2.4]-heptan-5,7-dion.

20 g (0,063 Mol) 1-[N-(3',5-dichlorphenyl)-carbonyloxy]-1-[(2,2)-dimethyl)-cyclopropancarbonsäure wurden 3 Stunden bei 100°C in 200 ml

Essigsäureanhydrid gerührt. Nach dem Abkühlen wurde das Reaktionsgemisch auf ca. 3 ℓ Eiswasser gegossen und das ausgefallene Produkt abfiltriert und gut mit Wasser gewaschen. Nach dem Trocknen erhielt man 18 g der Verbindung Nr. 2 vom Smp. 94-96°C.

Beispiel 2:

a) Herstellung des Zwischenproduktes

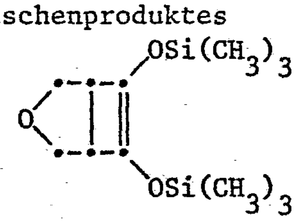

47,7 g (2,07 Mol) Natrium wurden in 450 ml Toluol pulverisiert. Dann wurde bei 100°C langsam ein Gemisch aus 106 g (0,49 Mol) Tetrahydrofuran-3,4-dicarbonsäurediäthylester und 237 g (2,19 Mol) Trimethylchlorsilan zugetropft und 16 Stunden bei 100°C Badtemperatur gerührt. Nach dem Abkühlen wurde filtriert und das Filtrat eingeengt. Bei der Destillation erhielt man 54,2 g Produkt vom Sdp. 109-117°C bei 29 mbar.

b) Herstellung des Zwischenproduktes

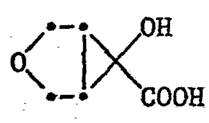

Zur Lösung von 54,4 g (0,2 Mol) des obigen Zwischenproduktes in 50 ml Pentan wurde bei -40°C eine Lösung von 32 g (0,2 Mol) Brom in 50 ml Pentan während 30 Minuten zugetropft. Dann wurde das Reaktionsgemisch auf ca. 500 ml 5 %ige Natronlauge gegossen und 2 Stunden verrührt. Die organische Phase wurde abgetrennt und die wässrige Lösung mit Kochsalz gesättigt. Dann wurde diese Lösung mit Aether extrahiert.

Die Aetherlösung wurde mit Natriumsulfat getrocknet, filtriert und eingeengt. Der Rückstand wurde mit wenig Aether verrieben, filtriert und getrocknet. Man erhielt so 13,3 g Produkt vom Smp. 135-137°C.

c) Herstellung des Zwischenproduktes

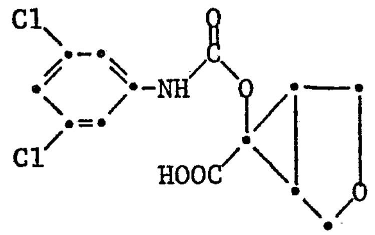

Zur Lösung von 7,2 g (0,05 Mol) des obigen Zwischenproduktes in 60 ml Aether wurde bei Raumtemperatur eine Lösung von 9,4 g (0,05 Mol) 3,5-Dichlorphenylisocyanat in 60 ml Toluol zugetropft. Das Reaktionsgemisch wurde dann 1 Tag bei Raumtemperatur gerührt und anschliessend filtriert. Der Rückstand ergab nach dem Anreiben mit Aether, Filtrieren und Trocknen 8,8 g Produkt.

d) Herstellung des Endproduktes:

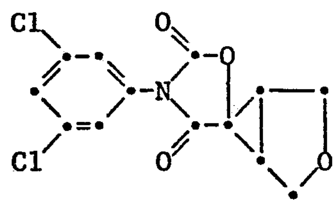

Verb. Nr. 15

8,8 g (0,026 Mol) des obigen Zwischenproduktes c) werden 3 Stunden bei 100°C in 100 ml Essigsäureanhydrid gerührt. Nach dem Abkühlen wurde eingeengt und der Rückstand mit Wasser verrieben. Nach dem Filtrieren und Trocknen erhielt man 4,2 g Produkt vom Smp. 191-192°C.

Auf analoge Weise oder nach einer der hierin beschriebenen Methoden können folgende Verbindungen der Formel I hergestellt werden:

Tabelle 1

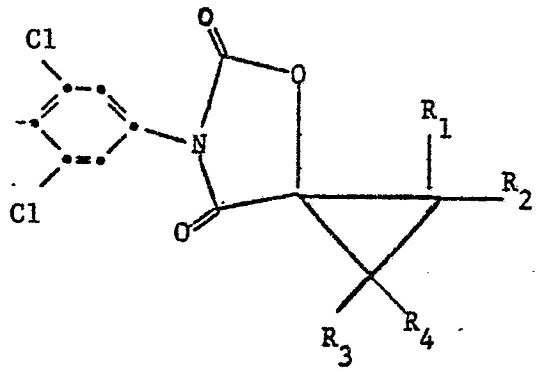

(I)

| Verb. Nr. | $R_1$ | $R_2$ | $R_3$ | $R_4$ | Physikal. Daten |
|---|---|---|---|---|---|
| 1 | $CH_3$ | H | H | H | Smp.122-124°C |
| 2 | $CH_3$ | $CH_3$ | H | H | Smp. 94-96°C |
| 3 | $CH_3$ | H | $CH_3$ | H | Smp. 90-91°C |
| 4 | $CH_3$ | $C_2H_5$ | H | H | Smp. 89-91°C |
| 5 | $CH_3$ | $CH_3$ | $CH_3$ | $CH_3$ | Smp.128-132°C |
| 6 | $C_3H_7$(n) | H | H | H | Smp.124-126°C |
| 7 | $C_3H_7$(i) | H | H | H | Smp.117-121°C |
| 8 | $C_4H_9$(n) | H | H | H | viscos |
| 9 | $C_4H_9$(t) | H | H | H | Smp.111-113°C |
| 10 | $CH_3$ | $CH_3$ | $CH_3$ | H | viscos |
| 11 | $C_2H_5$ | H | H | H | Smp.121-124°C |

Tabelle 2

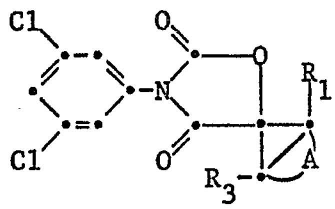

(I)

($R_1$ und $R_3$ = H)

| Verb. Nr. | A | Physik. Daten |
|---|---|---|
| 12 | $-(CH_2)_4-$ | zähes Oel |
| 13 | $-(CH_2)_3-$ | Oel |
| 14 | $-(CH_2)_2-$ | Oel |
| 15 | $-CH_2-O-CH_2-$ | Smp.191-192°C |

Formulierungsbeispiele

Beispiel 2

Stäubemittel: Zur Herstellung eines a) 5 %igen und b) 2 %igen Stäubemittels werden die folgenden Stoffe verwendet:

a) 5 Teile Wirkstoff No. 3
95 Teile Talkum

b) 2 Teile Wirkstoff No. 15
1 Teil hochdisperse Kieselsäure
97 Teile Talkum

Die Wirkstoffe werden mit den Trägerstoffen vermischt und vermahlen und können in dieser Form zur Anwendung verstäubt werden.

Beispiel 3

Granulat: Zur Herstellung eines 5 %igen Granulates werden die folgenden Stoffe verwendet:

| | |
|---|---|
| 5 | Teile eines der Wirkstoffe No. 1 bis 15 |
| 0,25 | Teile epoxydiertes Pflanzenöl |
| 0,25 | Teile Cetylpolyglykoläther |
| 3,50 | Teile Polyäthylenglykol |
| 91 | Teile Kaolin (Korngrösse 0,3 - 0,8 mm). |

Die Aktivsubstanz wird mit Epoxydiertem Pflanzenöl vermischt und mit 6 Teilen Aceton gelöst, hierauf wird Polyäthylenglykol und Cetylpolyglykoläther zugesetzt. Die so erhaltene Lösung wird auf Kaolin aufgesprüht, und anschliessend wird das Aceton im Vakuum verdampft. Ein derartiges Mikrogranulat wird vorteilhaft zur Bekämpfung von Bodenpilzen verwendet.

Beispiel 4:

Spritzpulver: Zur Herstellung eines a) 70 %igen, b) 40 %igen, c) und d) 25 %igen, e) 10 %igen Spritzpulvers werden folgende Bestandteile verwendet:

a)
- 70 Teile Wirkstoff No. 3
- 5 Teile Natriumdibutylnaphthylsulfonat
- 3 Teile Naphthalinsulfonsäuren-Phenolsulfonsäuren-Formaldehyd-Kondensat 3:2:1
- 10 Teile Kaolin
- 12 Teile Champagne-Kreide

b)
- 40 Teile Wirkstoff No. 15
- 5 Teile Ligninsulfonsäure-Natriumsalz
- 1 Teil Dibutylnaphthalinsulfonsäure-Natriumsalz
- 54 Teile Kieselsäure

c)
- 25 Teile Wirkstoff No. 2
- 4,5 Teile Calcium-Ligninsulfonat
- 1,9 Teile Champagne-Kreide/Hydroxyäthylcellulose-Gemisch 1:1
- 1,5 Teile Natrium-dibutyl-naphthalinsulfonat
- 10,5 Teile Kieselsäure
- 19,5 Teile Champagne-Kreide
- 28,1 Teile Kaolin

d) 25 Teile Wirkstoff No. 3
2,5 Teile Isooctylphenoxy-polyoxyäthylen-äthanol
1,7 Teile Champagne-Kreide/Hydroxyäthylcellulose-Gemisch 1:1
8,3 Teile Natriumaluminiumsilika
16,5 Teile Kieselgur
46 Teile Kaolin

e) 10 Teile Wirkstoff No. 15
3 Teile Gemisch der Natriumsalze von gesättigten Fettalkoholsulfaten
5 Teile Naphthalinsulfonsäure/Formaldehyd-Kondensat
82 Teile Kaolin.

Die Wirkstoffe werden in geeigneten Mischern mit den Zuschlagstoffen innig vermischt und auf entsprechenden Mühlen und Walzen vermahlen. Man erhält Spritzpulver von vorzüglicher Benetzbarkeit und Schwebefähigkeit, die sich mit Wasser zu Suspensionen der gewünschten Konzentration verdünnen und insbesondere zur Blattapplikation verwenden lassen.

Beispiel 5:

Emulgierbare Konzentrate: Zur Herstellung eines 25%igen emulgierbaren Konzentrates werden folgende Stoffe verwendet:

25 Teile eines der Wirkstoffe No. 1 bis 15
2,5 Teile epoxydiertes Pflanzenöl
10 Teile eines Alkylarylsulfonat/Fettalkoholpolyglykoläther-Gemisches
5 Teile Dimethylformamid
57,5 Teile Xylol

Aus solchen Konzentrationen können durch Verdünnen mit Wasser Emulsionen der gewünschten Anwendungskonzentration hergestellt werden, die besonders zur Blattapplikation geeignet sind.

Biologische Beispiele

Beispiel 6:

Wirkung gegen Botrytis cinerea auf Bohnen

a.) Residual-protektive Wirkung

Ca. 10 cm hohe Vicia-Pflanzen wurden mit einer aus Spritzpulver des Wirkstoffes hergestellten Spritzbrühe (0,02% Aktivsubstanz) besprüht. Nach 48 Stunden wurden die behandelten Pflanzen mit einer Konidiensuspension des Pilzes infiziert. Nach einer Inkubation der infizierten Pflanzen während 2-3 Tagen bei 95-100% relativer Luftfeuchtigkeit und 21°C erfolgte die Beurteilung des Pilzbefalls.

b.) Systemische Wirkung

Zu ca. 10 cm hohen Vicia-Pflanzen wurde eine aus Spritzpulver des Wirkstoffes hergestellte Spritzbrühe gegossen (0,006%) Aktivsubstanz bezogen auf das Bodenvolumen). Es wurde dabei darauf geachtet, dass die Spritzbrühe nicht mit den oberirdischen Pflanzenteilen in Berührung kam. Nach 48 Stunden wurden die behandelten Pflanzen mit einer Konidiensuspension des Pilzes infiziert. Die Beurteilung des Pilzbefalls erfolgte nach einer Inkubation der infizierten Pflanzen während 2-3 Tagen bei 95-100% relativer Luftfeuchigkeit und 21°C.

Mit den Verbindungen Nr. 1-15 wurde im Test a) der Pilzbefall vollständig verhindert. Im Test b) wurde der Pilzbefall mit den Verbindungen Nr. 1, 2, 3 und 15 verhütet.

Beispiel 7:

Wirkung gegen Rhizoctonia solani an Baumwolle

a.) Wirkung nach Bodenapplikation

Der Pilz wurde auf sterilen Hirsekörnern kultiviert und einer Erde-Sand-Mischung beigegeben. Die infizierte Erde wurde in Schalen abgefüllt und mit Baumwollsamen besät. Gleich nach der Aussaat wurde das als Spritzpulver formulierte Versuchspräparat als wässerige Suspension über die Erde

gegossen (20 ppm Aktivsubstanz bezogen auf das Erdvolumen). Die Erdschalen wurden darauf während 2-3 Wochen bei ca. 24°C im Gewächshaus aufgestellt und durch leichtes Ueberbrausen stets gleichmässig feucht gehalten. Bei der Auswertung der Tests wurde der Auflauf der Baumwollpflanzen bestimmt.

Mit den Verbindungen Nr. 3 und 15 wurde das Auflaufen von über 90 % der Pflanzen erzielt. Das entspricht dem Kontrollversuch mit nichtinfizierter Erde.

Beispiel 9: Residual-protektive Wirkung gegen Erysiphe graminis auf Gerste

Ca 8 cm hohe Gerstenpflanzen wurden mit einer aus Spritzpulver des Wirkstoffs hergestellten Spritzbrühe (0,02 % Aktivsubstanz) besprüht. Nach 3-4 Stunden wurden die behandelten Pflanzen mit Konidien des Pilzes bestäubt. Die infizierten Gerstenpflanzen wurden in einem Gewächshaus bei ca. 22°C aufgestellt. Die Ausbreitung des Pilzbefalls wurde nach 10 Tagen beurteilt. Im Vergleich zu Kontrollpflanzen wurde der Pilzbefall nach Behandlung mit den Verbindungen Nr. 4 und 15 vollständig zurückgedrängt.

Patentansprüche

1. Eine Verbindung der Formel

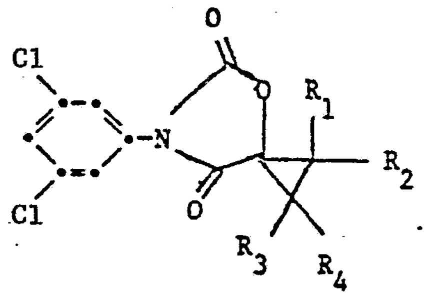

(I)

worin mindestens einer der Substituenten $R_1$, $R_2$, $R_3$, $R_4$ $C_1$-$C_4$-Alkyl ist und die anderen unabhängig voneinander Wasserstoff oder $C_1$-$C_4$-Alkyl bedeuten oder, falls $R_1$ und $R_3$ gleichzeitig Wasserstoff bedeuten, $R_2$ und $R_4$ gegebenenfalls eine $C_2$-$C_4$-Alkylenbrücke bilden, in der ein $CH_2$-Glied auch durch Sauerstoff ersetzt sein kann.

2. Eine Verbindung der Formel I gemäss Anspruch 1, worin $R_1$ bis $R_4$ im Umfang des Anspruchs 1 Wasserstoff oder Methyl bedeuten.

3. 4-Oxa-6-aza-6(3',5'-dichlorphenyl)-1-methyl-spiro[2.4]-heptan-5,7-dion gemäss Anspruch 1.

4. 4-Oxa-6-aza-6(3',5'-dichlorphenyl)-1,1-dimethyl-spiro[2.4]-heptan-5,7-dion gemäss Anspruch 1.

5. 4-Oxa-6-aza-6(3',5'-dichlorphenyl)-1,2-dimethyl-spiro[2.4]-heptan-5,7-dion gemäss Anspruch 1.

6. Die Verbindung der Formel

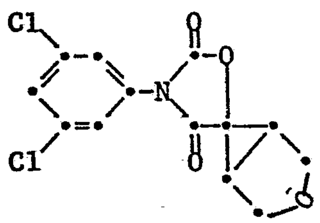

7. Verfahren zur Herstellung von Verbindungen der Formel I des Anspruchs 1, gekennzeichnet durch Cyclisierung einer Verbindung der Formel IV

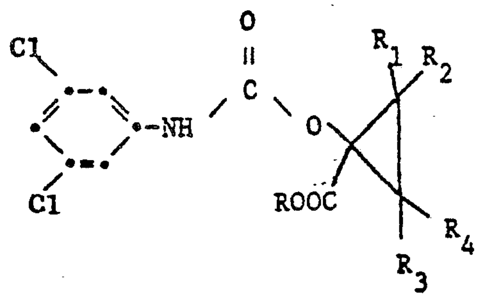

worin $R_1$ bis $R_4$ die für Formel I gegebene Bedeutung haben und R für Alkyl steht, im Temperaturbereich von -10° bis 160°C.

8. Verfahren gemäss Anspruch 7, dadurch gekennzeichnet, dass Verbindungen gemäss Anspruch 2 hergestellt werden.

9. Mikrobizides Mittel enthaltend als Wirkstoff eine Verbindung der Formel I gemäss Anspruch 1, zusammen mit mindestens einem geeigneten Trägermaterial und/oder oberflächenaktiven Mittel.

10. Mittel gemäss Anspruch 9, enthaltend als Wirkstoff eine Verbindung gemäss einem der Ansprüche 2 bis 5.

11. Mittel gemäss Anspruch 9, enthaltend die Verbindung gemäss Anspruch 6.

12. Verwendung einer Verbindung gemäss einem der Ansprüche 1 bis 6 zur Bekämpfung bzw. Verhütung eines Befalls durch phytopathogene Mikroorganismen.

0022086

Europäisches Patentamt

# EUROPÄISCHER RECHERCHENBERICHT

Nummer der Anmeldung
EP 80 81 0208

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| D | AGRICULTURAL AND BIOLOGICAL CHEMISTRY, Band 35, Nr. 11, November 1971, Seiten 1707-1719 Tokyo, JP. A. FUJINAMI et al.: "Studies on biological activity of cyclic imide compounds. Part I. Antimicrobial activity of 3-phenyloxazolidine-2,4-diones and related compounds" * Seite 1712, Tabelle V, Nr. 73 * | 1-12 |
| | GB - A - 982 940 (REPUBLIC OF FRANCE) * Seite 1, rechte Spalte, Zeilen 53-59; Seite 2, linke Spalte, Zeilen 11-25; Seiten 12,13; Ansprüche * | 1-12 |
| P | EP - A - 0 003 520 (BAYER A.G.) * Insgesamt * | 1-12 |
| E | EP - A - 0 012 261 (BAYER A.G.) * Insgesamt * | 1-12 |

KLASSIFIKATION DER ANMELDUNG (Int. Cl.3)

C 07 D 263/52
498/10
A 01 N 43/76
43/90
C 07 C 103/737
C 07 F 7/18
C 07 D 307/93
(C 07 D 498/10
307/00
263/00)

RECHERCHIERTE SACHGEBIETE (Int. Cl.3)

C 07 D 263/52
498/10
A 01 N 43/76
43/90

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Grunden angeführtes Dokument
&: Mitglied der gleichen Patentfamilie, übereinstimmendes Dokument

Der vorliegende Recherchenbericht wurde für alle Patentansprüche erstellt.

| Recherchenort | Abschlußdatum der Recherche | Prüfer |
|---|---|---|
| Den Haag | 22-09-1980 | ALLARD |

EPA form 1503.1 06.78